# EUROPEAN PATENT SPECIFICATION

(11) **EP 4 400 155 B1**
(45) Date of publication and mention of the grant of the patent: **25.06.2025**
(21) Application number: 23151603.0
(22) Date of filing: 13.01.2023
(51) Int. Cl.: A61N 1/04, A61N 1/32, A61N 1/40, A61N 2/00, A61N 2/02

(54) **MEDICAL SYSTEM FOR APPLYING A MICROCURRENT AND/OR A MAGNETIC FIELD TO A SUBJECT**
MEDIZINISCHES SYSTEM ZUR ANWENDUNG EINES MIKROSTROMS UND/ODER EINES MAGNETFELDES AUF EINE PERSON
SYSTÈME MÉDICAL POUR APPLIQUER UN MICROCOURANT ET/OU UN CHAMP MAGNÉTIQUE À UN SUJET

(43) Date of publication of application: 17.07.2024
(73) Proprietor: Azyro SA, 2339 Luxembourg (LU)
(72) Inventor: SERVAES, Jan, 8570 Vichte (BE); BENKHAI, Hicham, 86391 Stadtbergen (DE)
(74) Representative: Maiwald GmbH

(56) References cited:
- WO-A1-2016/038477
- FR-A1- 2 585 579
- IT-A1- UB20 159 823

## Description

### FIELD OF THE INVENTION

The present invention relates to a medical system for applying a microcurrent and/or a magnetic field to a subject.

### BACKGROUND OF THE INVENTION

In a subject's body, i.e., a human or animal body, some biological processes are known to be linked to electricity. For example, the functional basis of sensory, nerve and muscle cells is based on the generation, transmission and processing of electrical impulses containing information. By way of example, the beating of the heart is triggered by electrical impulses, the control of muscles occurs through electrical signals, and cognitive activity is related to brain currents. Thereby, a blood vessel, for example, may act as a transmission line to conduct electricity.

Further, it has been found that different electrical models can be established for tissue, bone, nerve, proteins, etc. For example, proteins may act as a semiconductor, tissues and bones may act as crystalline arrays, and nerves and muscles may act as electrical conductors, which conduct electromagnetic signals. At cell level, due to given permeability and transport properties of a cell membrane, an uneven ion distribution and thus charge distribution between a cell interior and a cell exterior is maintained, resulting in a membrane potential. In this regard, it has been found that the cell membrane may be described by an electrical model, in which the cell membrane model includes various ionic conductance and electromotive forces in parallel with a capacitor.

It is well known that diseases, particularly if they are triggered by pathogens, such as bacteria, parasites, fungi, viruses, etc., in the human or animal body are based at least in part on structures, such as proteins, DNA, cells, etc., which may be electrically and/or electromagnetically influenced or at least stimulated by externally applied electric and/or electromagnetic emissions.

Just like diseases, healing of a wounded skin or other of types of skin diseases of a human or animal can be promoted by the application of microcurrents to the wounded skin, and thus offers a unique treatment option to help healing complicated and recalcitrant wounds.

In addition, the application of a low power magnetic field is reported to be beneficial to skin healing, and further reduces the risk of wound infection.

In the prior art, WO 2016/038477 A1 describes a skin patch for cosmetic or medical purposes having several integrated electrodes capable of generating microcurrents.

ITUB 20159823A1 describes a medical device including a plurality of electrical electrodes and a magnetic coil. The electrodes are connected to a pulsed electric field generator and are intended to come in contact with the patient's skin in the area of the body to be treated to transmit current impulses. The magnetic coil is connected to a pulsed magnetic field generator in order to subject the area of the body to be treated with magnetic field pulses. The magnetic coil is mounted within a chamber of a handpiece of the medical device.

### SUMMARY OF THE INVENTION

Therefore, a need exists for providing a skin patch or wound dressing that is simple to use and does not require the assistance of professional medical care, yet provides the benefits of external electric and/or magnetic tissue stimulation in order to promote healing, in particular wound or skin healing.

These and possibly other objects of the present invention are solved by the subject matter of independent claim 1. Optional or preferred features are subject of the dependent claims.

According to the invention, a medical system for applying a microcurrent and/or a magnetic field to a subject is provided. The medical system comprises a microcurrent-generating assembly having at least one pair of planar electrodes configured to directly contact a subject, a magnetic field-generating assembly having at least one electromagnetic coil, and engagement means configured for engaging the microcurrent-generating assembly and the magnetic field-generating assembly such that the pair of electrodes and the electromagnetic coil are spatially separated and electrically insulated from another. The medical system further comprises a non-continuous adhesive layer configured to adhesively attach the microcurrent-generating assembly to the subject.

Preferably, the microcurrent-generating assembly has an opening with a size adapted to a size of a wound or skin area to be treated, wherein the electrodes of the pair of electrodes are arranged at opposite sides of the opening.

In an embodiment, the pair of electrodes of the microcurrent-generating assembly includes an outer electrode and an inner electrode arranged within the outer electrode.

Preferably, the microcurrent-generating assembly has a layered structure and comprises two electromagnetic coils, wherein one electromagnetic coil in one layer spirals radially inwardly, and the other electromagnetic coil in another layer spirals radially outwardly, and wherein the two electromagnetic coils are electrically connected to one another.

More preferably, the adhesive layer is made of a biocompatible hydrogel material or a thermoplastic polyurethane.

Yet more preferably, the pair of electrodes are printed on the adhesive layer using PCB technology.

In an embodiment, the adhesive layer includes voids in which the pair of electrodes are arranged.

In a further embodiment, the at least one electromagnetic coil is a planar electromagnetic coil.

Preferably, the at least one electromagnetic coil is printed on an electrically insulating layer or on a transparent top layer using PCB technology.

More preferably, each electrode of the pair of electrodes and the at least one electromagnetic coil has terminal ends configured to be connected to a signal generator configured to electrically drive the pair of electrodes and the at least one electromagnetic coil.

In an embodiment, the medical system further comprises an ID chip programmed to allow for identification of the medical system.

In a further embodiment, the medical system further comprises a sensor having terminal ends configured to be connected to a datalogger.

Preferably, the microcurrent-generating assembly is configured to generate microcurrents in a range of 0 - 30 mA, preferably 0 - 10 mA, and wherein the magnetic field-generating assembly is configured to generate a magnetic field with a magnetic flux in a range of 0 - 30 mT, preferably 0 - 10 mT.

More preferably, the engagement means is configured such that a distance between the at least one electromagnetic coil and the patient is about 1 - 10 mm, preferably 2 mm.

Yet more preferably, the engagement means is a mechanical form-fitting linkage between the microcurrent-generating assembly and the magnetic field-generating assembly.

In an embodiment, the engagement means includes a collar provided on the microcurrent-generating assembly and an opening provided on the magnetic field-generating assembly, and wherein the opening is sized such that the collar of the microcurrent-generating assembly form-fittingly fits into the opening of the magnetic field-generating assembly.

In a further embodiment, the engagement means is a hinge that allows a pivotal movement of the magnetic field-generating assembly relative to the microcurrent-generating assembly.

Preferably, the hinge is a living hinge.

It is noted that the above embodiments may be freely combined with each other. These and other aspects of the present invention will become apparent from and will be elucidated below with reference to preferred embodiments of the invention described hereinafter.

### BRIEF DESCRIPTION OF THE DRAWINGS

Preferred embodiments of the invention will now be described by way of example with reference to the drawings.
Fig. 1 shows an exploded perspective view of a medical system for applying a microcurrent and/or a magnetic field to a subject according to a preferred embodiment of the invention;
Fig. 2 shows a top view and a vertical cross-section of the medical system of Fig. 1;
Fig. 3 shows a top view and a vertical cross-section of a medical system according to another preferred embodiment of the invention;
Fig. 4 shows schematically the magnetic field lines of the medical system of Fig. 1 and Fig. 2;
Fig. 5 shows a perspective view of a medical system according to yet another preferred embodiment of the invention;
Fig. 6 shows a top view of a medical system according to a further preferred embodiment of the invention;
Fig. 7 shows a top view of an alternative medical system according to yet a further preferred embodiment of the invention.

### DETAILED DESCRIPTION OF EMBODIMENTS

The present invention generally relates to a medical system for applying a microcurrent and/or a magnetic field to a subject. In particular, the medical system may be a skin patch or wound dressing that specifically applies microcurrents and magnetic fields to a certain skin area or a wounded skin of a patient. However, the medical system of the present invention may be more generally used for in-vivo influencing biological cellular material by applying specifically selected and finely controlled electric microcurrents and magnetic fields to the biological cellular material. For example, and without being limited, the medical system of the present invention may be used for healing various types of skin diseases, in particular healing of wounded skin as well as joint healing.

The application of a combination of an electric microcurrent and a magnetic field may be applied to the biological cellular material either at the same time or at different times. The electric microcurrent may be a direct current or an alternating current with a timely constant frequency and/or amplitude. The magnetic field may also be a constant magnetic field or an alternating magnetic field. Specifically, the amplitude, the frequency and/or the waveform of the electric microcurrent and/or the magnetic field may timely vary or may be timely constant. Preferably, the microcurrents are preferably in a range of 0 - 30 mA, more preferably 10 mA, and the magnetic field has preferably a magnetic flux in a range of 0 - 30 mT, more preferably 10 mT.

It is advantageous, if the medical system has a layered structure, where a first part of the layers forms a microcurrent-generating assembly, and where a second part of the layers forms a magnetic field-generating assembly.

With or without a layered structure, and in order to able to use the microcurrent-generating assembly by itself and independent from the magnetic field-generating assembly, both assemblies can releasably be engaged with one another by means of engagement means. The engagement means can be quite general and may be selected from the group consisting of form-fitting or force-fitting engagement means, such as an opening or recess in one assembly and a protruding collar on the other assembly, clamping means, screw-fitting means, or even adhesive engagement means.

The medical system of the present invention is preferably adhesive, more preferably self-adhesive which allows easy attachment of the medical system to the skin of a patient, without any specific skills in medical care. The holding force is preferably comparable to a classic conventional wound healing plaster, enabling application of the the medical system to intact skin of a patient for several days.

The medical system is preferably available in several standard sizes, depending on the size of the skin area or wound to be treated. All materials used for the medical system should preferably be biocompatible. The medical system will also be worn on curved body parts and should therefore preferably be bendable by up to more than 180° without causing discomfort to the patient or continuous excessive force on the adhesive parts of the medical system. A limited amount of stretchability, towards a spherical shape, will help to adapt to smaller or sphere-shaped body parts. The medical system should preferably be resilient to constant movement or muscle activity of the patient. No delamination under stress conditions due to bending or continuous movement or in an attempt to remove the medical system from the patient shall preferably occur. Nor shall electrically conductive parts become exposed and thus freely accessible.

The medical system should also avail itself to sterilization, for example by the use of Gamma rays or ethylene oxide.

The present invention will now be explained by way of example in terms of a wound healing device 10, although the medical system of the present invention is not limited to the use as a wound healing device. The wound healing device 10 may not only be applied to a wound, but may also be applied to certain skin areas in order to treat the skin or subcutaneous structures in that area of the human or animal body.

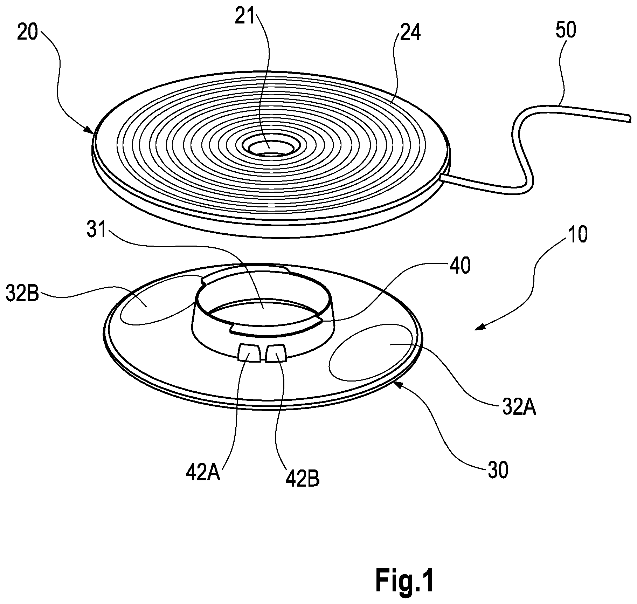

Fig. 1 shows an exploded perspective view of a wound healing device 10 according to a preferred embodiment of the invention. The wound healing device 10 includes a magnetic field-generating assembly 20, a microcurrent-generating assembly 30 and engagement means 40, here in the form of a collar 40 which is preferably provided on the microcurrent-generating assembly 30, and which is engageable with a central opening 21 or a circular recess or circular groove provided on or on a lower surface of the magnetic field-generating assembly 20.

The wound healing device 10 has preferably a circular shape, but may have other types of shapes as well.

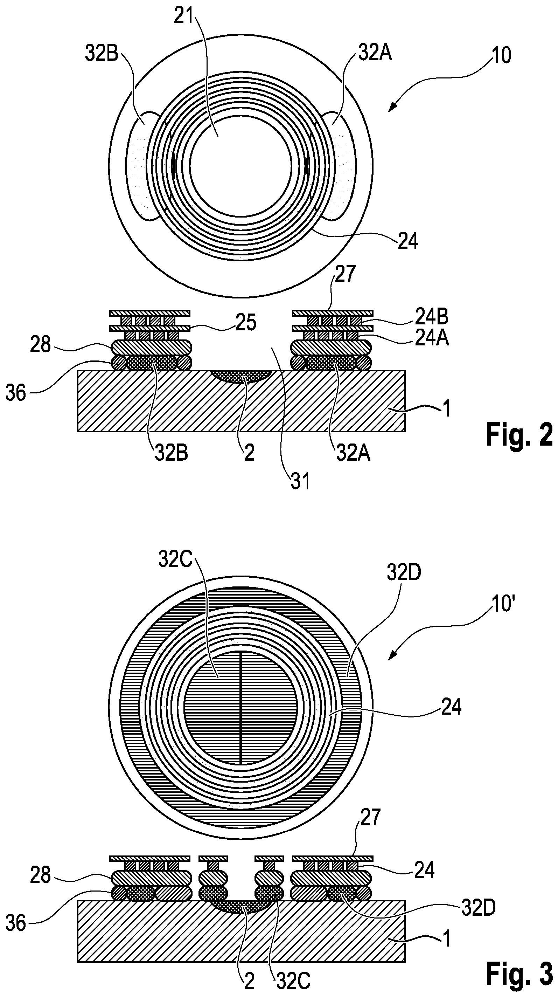

The magnetic field-generating assembly 20 has a preferably central opening 21, and the microcurrent-generating assembly 30 has a preferably central opening 31. The size of the opening 21 may be the same as the size of the opening 31, but may also be different. The opening 31 allows placement of the wound healing device 10 around the wound 2 (Fig. 2), leaving the wound surface untouched in order to allow a standard wound dressing to cover the actual wound 2. The wound dressing should ideally be changeable without having to remove the wound healing device 10 from the skin of the patient. The mesh size of the wound dressing should be selected such that wound exudate may penetrate the wound dressing.

The wound healing device 10 will contact the intact skin surrounding the wound 2. As shown in Fig. 1, the microcurrent-generating assembly 30 has two electrodes 12A, 12B preferably arranged opposite the opening 31.

Electrical contacts 42A, 42B are provided on the otherwise preferably electrically nonconductive collar 40, which establish an electrical connection of the electrodes 32A, 32B to the cable 50, when the microcurrent-generating assembly 30 is mounted together with the magnetic field-generating assembly 20. The cable 50 is used to connect the wound healing device 10 to an external signal generator 100 (Fig. 3) which is configured to generate the signals for producing the microcurrents and magnetic fields to be applied to the patient.

Fig. 2 shows a top view and a vertical cross-section of the wound healing device 10 of Fig. 1, however without the engagement means 40. In this embodiment, the size of the opening 21 is the same as the size of the opening 31.

The electrodes 32A, 32B are in direct contact with the skin of the patient in order to force microcurrents through the wound or skin surface. These electrodes 32A, 32B may be made from a biocompatible electrically conductive material, such as a metal, and may be embedded in voids of an adhesive layer 36 or may be positioned or printed on the surface thereof using PCB technology (printed circuit board technology). The adhesive layer 36 may be a self-adhesive layer 36. The adhesive layer 36 allows attachment of the wound healing device 10 to the skin of the patient. For example, the adhesive layer 36 may be a hydrogel material layer or a thermoplastic polyurethane.

A spacer layer 28 may optionally be provided on top of the electrodes 32A, 32B and the adhesive layer 36. The thickness of the spacer layer 28 may be selected such that the distance between a magnetic coil 24A and the skin of the patient is preferably in a range of 1 - 10 mm, more preferably is about 2 mm. This distance may vary upon need and serves the purpose to allow penetration of the magnetic field generated by the magnetic coil 24A by a certain amount beneath the skin or wound of the patient.

The spacer layer 28 may, however, be omitted as the engagement means 40 is configured such that when the microcurrent-generating assembly 30 is engaged with the magnetic field-generating assembly 20, there will be a gap between the microcurrent-generating assembly 30 and the magnetic field-generating assembly 20 that spatially separates and electrically insulates the microcurrent-generating assembly 30 from the magnetic field-generating assembly 20.

In a preferred embodiment, there may be more than one magnetic coil 24A. In Fig. 2, a second magnetic coil 24B is shown above the magnetic coil 24A. Between the two magnetic coils 24A, 24B is provided a substrate layer 25.

Although the magnetic coils 24A, 24B are shown as concentric rings in Fig. 1 and Fig. 2 (as well as Fig. 3), the magnetic coils 24A, 24B are indeed formed as a spiral or helix, preferably as a planar spiral. "Planar" in this context refers to the way the magnetic coils 24A, 24B are wound, rather than to the overall geometry of the magnetic coils 24A, 24B being flat like the water surface. Although the magnetic coils 24A, 24B being planar, they may bend and adopt a curved shape. In a preferred embodiment, the electromagnetic coil 24A may preferably spiral radially inwardly, and the electromagnetic coil 24B may preferably spiral radially outwardly, wherein the two electromagnetic coils 24A, 24B are electrically connected to one another.

The electromagnetic coils 24A, 24B may preferably be printed onto the substrate layer 25 using PCB technology.

There may also be an uppermost layer 27 that is preferably transparent and shields the electromagnetic coils 24A, 24B and the electrodes 32A, 32B from any electromagnetic impact or dirt and dust particles of the environment. This top layer 27 has preferably printed information on it which during wound or skin healing allows monitoring the progress of the healing process. The information printed on the top layer 27 may also include information as regards the type and individual serial number of the wound healing device 10, for example in the form of a QR code, markings to determine size and orientation of the wound healing device 10, and/or color references for comparison with the wound color and for electronic detection. Photographs may be taken of the top layer 27 in frequent intervals, allowing the wound healing process to be monitored. Artificial intelligence may be used for this purpose.

The wound healing device 10 may also include an ID chip (not shown) for the purpose of electronic identification and authentication of the wound healing device 10. Identification and authentication of the wound healing device 10 ensures safe and simple use of the wound healing device 10 by an ordinary person. In addition, it ensures that the correct wound healing device 10 is used for the right treatment program and may further help preventing re-use of the disposable wound healing device 10.

The wound healing device 10 is preferably connected to a signal generator 100 (Fig. 3), via the cable 50 (Fig. 1) or wirelessly in order to provide the stimuli to the electrodes 32A, 32B and the electromagnetic coils 24A, 24B. To this end, the wound healing device 10 has preferably several electrical contacts for the signal generator 100, that is two contacts for the electrodes 32A, 32B, two contacts for each electromagnetic coil 24A, 24B, two contacts for the ID chip that allows identification and authentication of the wound healing device 10 and two contacts for a datalogger that measures various physical and physiological parameters, such as humidity of the wound, skin temperature, etc. The sensors needed for that purpose are attached to the wound healing device 10 and are not shown in Fig. 1, Fig. 2 or Fig.3.

The size of the wound healing device 10 is preferably about 8 cm in diameter, the electrodes 32A, 32B preferably have a size of 5 cm by 1.2 cm, and the opening 31 preferably has a diameter of 4 cm. Preferably, each electromagnetic coil 24A, 24B may have ten to thirty windings, such as for example 16, and the total width of each electromagnetic coil 24A, 24B may be in a range of 1 cm to 3 cm. The total resistance of each electromagnetic coil 24A, 24B should preferably be below 1 Ohm.

Fig. 3 shows another preferred embodiment of the invention, again without the engagement means 40. The same reference numerals used in Fig. 1 and Fig. 2 denote the same or at least functionally similar or identical parts.

The main difference between the wound healing device 10 of Fig. 2 and the wound healing device 10' of Fig. 3 is that there is no opening 31 in the centre of the microcurrent-generating assembly 30. Instead, the microcurrent-generating assembly 30 of the wound healing device 10' has a central electrode 32C and a ring-shaped electrode 32D in a radial distance from the central electrode 32C. There is a microcurrent flow between the central electrode 32C and the ring-shaped electrode 32D. The central electrode 32C may be placed on top of the wound and therefore directly touches the wound. It may be sized depending on the size of the wound or a particular skin area to be treated. The central electrode 32C may have small openings through which exudate of the wound can penetrate. The ring-shaped electrode 32D may have an over all shape of a closed or open ring, that is circular, oval, square, rectangular or even polynomial, with a multiplicity of spirals forming the ring-shaped electrode 32D.

Although the vertical cross-section of Fig. 3 shows only one electromagnetic coil 24, there may be more than one, for example two electromagnetic coils, such as was the case in the wound healing device 10 of Fig. 2.

Preferably, no adhesive layer 36 is provided in the central part of the wound healing device 10', but instead only radially ouwardly and around the circumference of the ring-shaped electrode 32D.

A spacer layer 28 is provided on top of the electrodes 32C, 32D and the adhesive layer 36, and radially inwardly and around the circumference of the ring-shaped electrode 32D. The thickness of the spacer layer 28 is selected such that the distance between the magnetic coil 24 and the skin of the patient is preferably in a range of 1 - 10 mm, more preferably about 2 mm. This distance may vary upon need and serves the purpose to allow penetration of the magnetic flux generated by the magnetic coil 24 by a certain amount beneath the skin or wound of the patient.

The spacer layer 28 may, however, be omitted as the engagement means 40 is configured such that when the microcurrent-generating assembly 30 is engaged with the magnetic field-generating assembly 20, there will be a gap between the microcurrent-generating assembly 30 and the magnetic field-generating assembly 20 that spatially separates and electrically insulates the microcurrent-generating assembly 30 from the magnetic field-generating assembly 20.

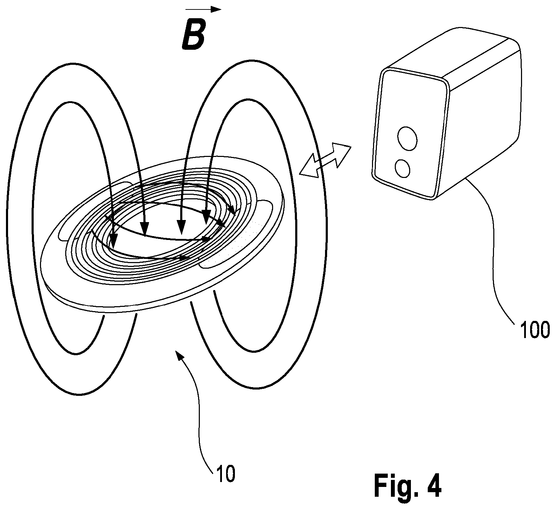

Fig. 4 shows the schematically the magnetic field lines of the wound healing device 10 of Fig. 2 and the wound healing device 10' of Fig. 3. As mentioned before, the distance between the electromagnetic coil 24A (or 24) may be selected such that the distance between the electromagnetic coil 24A (or 24) and the skin of the patient is preferably in range of 1 mm to 10 mm, more preferably about 2 mm. If more than one electromagnetic coil 24 is used, such as electromagnetic coils 24A and 24B, the distance between the lowest electromagnetic coil 24A and the skin is preferably in range of 1 mm to 10 mm, more preferably about 2 mm.

Fig. 4 also indicates that the wound healing device 10, 10' is to be connected to a signal generator 100 which provides the stimuli to the electrodes 32A, 32B or 32C, 32D and the electromagnetic coil 24 or electromagnetic coils 24A and 24B in order to allow treatment of diseased skin (or diseased subcutaneous structures, such as joint disease) by the application of microcurrents and magnetic fields. The signal generator 100 may also be named as a current generator as it is capable of generating the currents needed in the microcurrent-generating assembly 30 and the magnetic field-generating assembly 20.

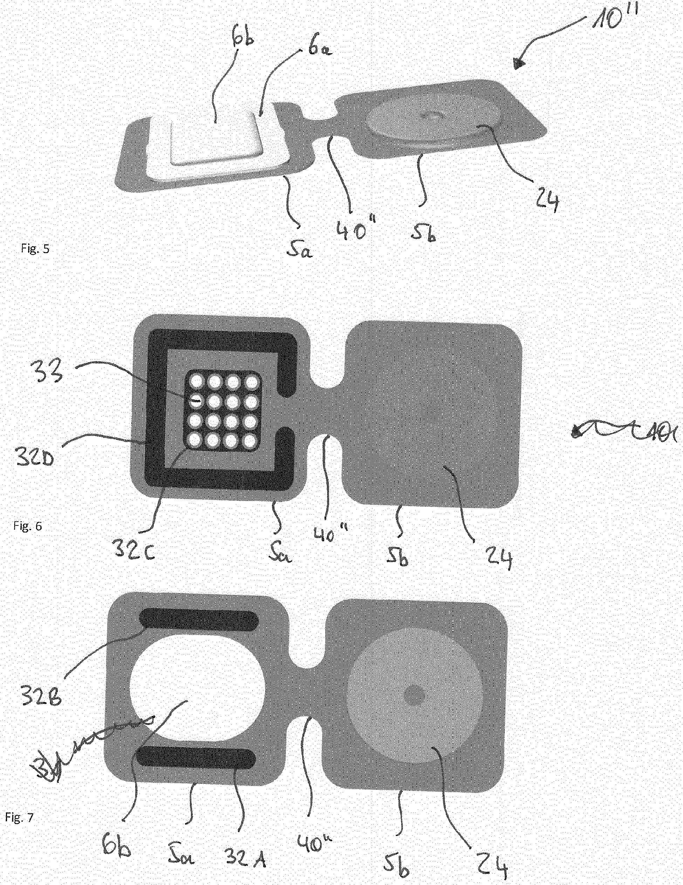

Fig. 5 shows a schematic perspective view of a medical system 10" according to yet another preferred embodiment of the invention. In this embodiment, the magnetic field-generating assembly 20 with the magentic coil 24 is hinged to the microcurrent-generating assembly 30 (not shown) via a hinge 40". The hinge is preferably a living hinge 40". Both the microcurrent-generating assembly 30 and the magnetic field-generating assembly 20 are preferably placed onto an adhesive foil 5a, 5b that is used to attach the medical system 10" to the wound or skin area of a subject. A wound dressing 6a is placed between the microcurrent-generating assembly 30 and the magnetic field-generating assembly 20, with a padded are 6b of the wound dressing 6a covering a central area of the microcurrent-generating assembly 30.

In the embodiment of Fig. 6, the medical system 10" has an electrode arrangement as that of the embodiment of Fig. 3, with an outer electrode 32D and an inner electrode 32C. The outer electrode 32D may be a closed or open ring or structure, that is circular, oval, square, rectangular or even polynomial, with a multiplicity of spirals forming the ring-shaped electrode 32D. The inner or central electrode 32C may have small openings 33 through which exudate of the wound can penetrate. The wound dressing may be placed directly onto the skin of the subject and underneath of the inner electrode 32C. Alternatively, it may also be placed between the microcurrent-generating assembly 30 and the magnetic field-generating assembly 20. In use, the magnetic field-generating assembly 20 may be pivoted about the hinge 40" to basically rest on the microcurrent-generating assembly 30, with a certain distance between the two assemblies 20, 30. The adhesive foil 5a, 5b is used to attach the medical system 10" to the skin of the subject.

In the embodiment of Fig. 7, the medical system 10" has an electrode arrangement as that of the embodiment of Fig. 2, with two electrodes 32A, 32B being positioned on opposite sides of opening 31 (not shown). A wound dressing, in particular the padded part 6b od the wound dressing 6a, may be placed directly onto the skin of the subject and underneath the microcurrent-generating assembly 30 and aligned with the opening 31. Alternatively, it may also be placed between the microcurrent-generating assembly 30 and the magnetic field-generating assembly 20. In use, the magnetic field-generating assembly 20 may be pivoted about the hinge 40" to basically rest on the microcurrent-generating assembly 30, with a certain distance between the two assemblies 20, 30. Again, the adhesive foil 5a, 5b is used to attach the medical system 10" to the skin of the subject.

## Claims

1. A medical system (10) for applying a microcurrent and/or a magnetic field to a subject, comprising:
a microcurrent-generating assembly (30) having at least one pair of planar electrodes (32A, 32B) configured to directly contact a subject;
a magnetic field-generating assembly (20) having at least one electromagnetic coil (24); and
engagement means (40) configured for engaging the microcurrent-generating assembly (30) and the magnetic field-generating assembly (20) such that the pair of electrodes (32A, 32B) and the electromagnetic coil (24) are spatially separated and electrically insulated from another,
**characterized by** further comprising a non-continuous adhesive layer (36) configured to adhesively attach the microcurrent-generating assembly (30) to the subject.

2. The medical system (10) of claim 1, wherein the microcurrent-generating assembly (30) has an opening (31) with a size adapted to a size of a wound or skin area (2) to be treated, and wherein the electrodes (32A, 32B) of the pair of electrodes are arranged at opposite sides of the opening (31).

3. The medical system (10) of claim 1, wherein the pair of electrodes of the microcurrent-generating assembly (30) includes an outer electrode (32D) and an inner electrode (32C) arranged within the outer electrode (32D).

4. The medical system (10) of any of the preceding claims, wherein the magnetic field-generating assembly (20) has a layered structure and comprises two electromagnetic coils (24A, 24B), and wherein one electromagnetic coil (24A) in one layer spirals radially inwardly, and the other electromagnetic coil (24B) in another layer spirals radially outwardly, and wherein the two electromagnetic coils (24A, 24B) are electrically connected to one another.

5. The medical system (10) of claim 1, wherein the adhesive layer (36) is made of a biocompatible hydrogel material or a thermoplastic polyurethane.

6. The medical system (10) of claim 1 or 5, wherein the pair of electrodes (32A, 32B) are printed on the adhesive layer (36) using PCB technology.

7. The medical system (10) of claim 1 or 5, wherein the adhesive layer (36) includes voids in which the pair of electrodes (32A, 32B) are arranged.

8. The medical system (10) of any of the preceding claims, wherein the at least one electromagnetic coil (24) is a planar electromagnetic coil.

9. The medical system (10) of any of the preceding claims, wherein the at least one electromagnetic coil (24) is printed on an electrically insulating layer (25) or on a transparent top layer (27) using PCB technology.

10. The medical system (10) of any of the preceding claims, wherein each electrode (32A, 32B) of the pair of electrodes and the at least one electromagnetic coil (24) has terminal ends configured to be connected to a signal generator (100) configured to electrically drive the pair of electrodes (32A, 32B) and the at least one electromagnetic coil (24).

11. The medical system (10) of any of the preceding claims, further comprising an ID chip programmed to allow for identification of the medical system (10).

12. The medical system (10) of any of the preceding claims, further comprising a sensor having terminal ends configured to be connected to a datalogger.

13. The medical system (10) of any of the preceding claims, wherein the microcurrent-generating assembly (30) is configured to generate microcurrents in a range of 0 - 30 mA, preferably 0 - 10 mA, and wherein the magnetic field-generating assembly (20) is configured to generate a magnetic field with a magnetic flux in a range of 0 - 30 mT, preferably 0 - 10 mT.

14. The medical system (10) of any of the preceding claims, wherein the engagement means (40) is configured such that a distance between the at least one electromagnetic coil (24) and the patient is in a range of 1- 10 mm, preferably 2 mm.

15. The medical system (10) of any of the preceding claims, wherein the engagement means (40) is a mechanical form-fitting linkage between the microcurrent-generating assembly (30) and the magnetic field-generating assembly (20).

16. The medical system (10) of any of the preceding claims, wherein the engagement means includes a collar (40) provided on the microcurrent-generating assembly (30) and an opening (21) provided on the magnetic field-generating assembly (20), and wherein the opening (21) is sized such that the collar (40) of the microcurrent-generating assembly (30) form-fittingly fits into the opening (21) of the magnetic field-generating assembly (20).

17. The medical system (10) of any of claims 1 to 14, wherein the engagement means is a hinge (40') that allows a pivotal movement of the magnetic field-generating assembly (20) relative to the microcurrent-generating assembly (30).

18. The medical system (10) of claim 17, wherein the hinge (40') is a living hinge.

## Patentansprüche

1. Medizinisches System (10) zum Anlegen eines Mikrostroms und/oder eines Magnetfelds an einer Person, aufweisend
eine mikrostromerzeugende Anordnung (30) mit mindestens einem Paar planarer Elektroden (32A, 32B), die eingerichtet sind, in direktem Kontakt mit einer Person zu stehen;
eine magnetfelderzeugende Anordnung (20) mit mindestens einer elektromagnetischen Spule (24); und
Eingriffsmittel (40), die eingerichtet sind, die mikrostromerzeugende Anordnung (30) und die magnetfelderzeugende Anordnung (20) so in Eingriff zu bringen, dass das Elektrodenpaar (32A, 32B) und die elektromagnetische Spule (24) räumlich voneinander getrennt und elektrisch isoliert sind,
**dadurch gekennzeichnet, dass** es ferner eine nicht kontinuierliche Klebeschicht (36) aufweist, die eingerichtet ist, die mikrostromerzeugende Anordnung (30) an der Person klebend zu befestigen.

2. Medizinisches System (10) nach Anspruch 1, wobei die mikrostromerzeugende Anordnung (30) eine Öffnung (31) mit einer Größe aufweist, die an die Größe einer zu behandelnden Wunde oder Hautfläche (2) angepasst ist, und wobei die Elektroden (32A, 32B) des Elektrodenpaares an gegenüberliegenden Seiten der Öffnung (31) angeordnet sind.

3. Medizinisches System (10) nach Anspruch 1, wobei das Elektrodenpaar der mikrostromerzeugenden Anordnung (30) eine äußere Elektrode (32D) und eine innere Elektrode (32C) aufweist, die innerhalb der äußeren Elektrode (32D) angeordnet ist.

4. Medizinisches System (10) nach einem der vorhergehenden Ansprüche, wobei die magnetfelderzeugende Anordnung (20) eine Schichtstruktur aufweist und zwei elektromagnetische Spulen (24A, 24B) aufweist, und wobei eine elektromagnetische Spule (24A) in einer Schicht radial nach innen spiralförmig ist und die andere elektromagnetische Spule (24B) in einer anderen Schicht radial nach außen spiralförmig ist, und wobei die beiden elektromagnetischen Spulen (24A, 24B) elektrisch miteinander verbunden sind.

5. Medizinisches System (10) nach Anspruch 1, wobei die Klebeschicht (36) aus einem biokompatiblen Hydrogelmaterial oder einem thermoplastischen Polyurethan hergestellt ist.

6. Medizinisches System (10) nach Anspruch 1 oder 5, wobei das Elektrodenpaar (32A, 32B) mit Hilfe der Leiterplattentechnologie auf die Klebeschicht (36) gedruckt wird.

7. Medizinisches System (10) nach Anspruch 1 oder 5, wobei die Klebeschicht (36) Hohlräume enthält, in denen das Elektrodenpaar (32A, 32B) angeordnet ist.

8. Medizinisches System (10) nach einem der vorhergehenden Ansprüche, wobei die mindestens eine elektromagnetische Spule (24) eine planare elektromagnetische Spule ist.

9. Medizinisches System (10) nach einem der vorhergehenden Ansprüche, wobei die mindestens eine elektromagnetische Spule (24) auf eine elektrisch isolierende Schicht (25) oder auf eine transparente Deckschicht (27) in Leiterplattentechnik gedruckt ist.

10. Medizinisches System (10) nach einem der vorhergehenden Ansprüche, wobei jede Elektrode (32A, 32B) des Elektrodenpaares und die mindestens eine elektromagnetische Spule (24) Anschlussenden aufweisen, die eingerichtet sind, mit einem Signalgenerator (100) verbunden zu werden, der eingerichtet ist, das Elektrodenpaar (32A, 32B) und die mindestens eine elektromagnetische Spule (24) elektrisch anzusteuern.

11. Medizinisches System (10) nach einem der vorangehenden Ansprüche, ferner aufweisend einen ID-Chip, der programmiert ist, die Identifizierung des medizinischen Systems (10) zu ermöglichen.

12. Medizinisches System (10) nach einem der vorhergehenden Ansprüche, ferner aufweisend einen Sensor mit Anschlussenden, die für den Anschluss an einen Datenlogger konfiguriert sind.

13. Medizinisches System (10) nach einem der vorhergehenden Ansprüche, wobei die mikrostromerzeugende Anordnung (30) eingerichtet ist, Mikroströme in einem Bereich von 0 - 30 mA, vorzugsweise 0 - 10 mA, zu erzeugen, und wobei die magnetfelderzeugende Anordnung (20) eingerichtet ist, ein Magnetfeld mit einem Magnetfluss in einem Bereich von 0 - 30 mT, vorzugsweise 0 - 10 mT, zu erzeugen.

14. Medizinisches System (10) nach einem der vorhergehenden Ansprüche, wobei das Eingriffsmittel (40) so konfiguriert ist, dass ein Abstand zwischen der mindestens einen elektromagnetischen Spule (24) und dem Patienten in einem Bereich von 1 - 10 mm, vorzugsweise 2 mm, liegt.

15. Medizinisches System (10) nach einem der vorhergehenden Ansprüche, wobei das Eingriffsmittel (40) eine mechanische formschlüssige Verbindung zwischen der mikrostromerzeugenden Anordnung (30) und der magnetfelderzeugenden Anordnung (20) ist.

16. Medizinisches System (10) nach einem der vorhergehenden Ansprüche, wobei das Eingriffsmittel einen Kragen (40), der an der mikrostromerzeugenden Anordnung (30) vorgesehen ist, und eine Öffnung (21), die an der magnetfelderzeugenden Anordnung (20) vorgesehen ist, aufweist, und wobei die Öffnung (21) so bemessen ist, dass der Kragen (40) der mikrostromerzeugenden Anordnung (30) formschlüssig in die Öffnung (21) der magnetfelderzeugenden Anordnung (20) passt.

17. Medizinisches System (10) nach einem der Ansprüche 1 bis 14, wobei das Eingriffsmittel ein Gelenk (40') ist, das eine Schwenkbewegung der magnetfelderzeugenden Anordnung (20) relativ zur mikrostromerzeugenden Anordnung (30) ermöglicht.

18. Medizinisches System (10) nach Anspruch 17, wobei das Gelenk (40') ein lebendes Gelenk ist.

## Revendications

1. Système médical (10) permettant d'appliquer un micro-courant et/ou un champ magnétique à un sujet, comprenant :
un ensemble générateur de micro-courants (30) possédant au moins une paire d'électrodes planes (32A, 32B) configurées pour entrer directement en contact avec un sujet ;
un ensemble générateur de champ magnétique (20) possédant au moins une bobine électromagnétique (24) ; et
un moyen d'engagement (40) configuré pour engager l'ensemble générateur de micro-courants (30) et l'ensemble générateur de champ magnétique (20) de manière à ce que la paire d'électrodes (32A, 32B) et la bobine électromagnétique (24) soient séparées spatialement et isolées électriquement l'une de l'autre,
**caractérisé par** le fait de comprendre en outre une couche adhésive non continue (36) configurée pour attacher de manière adhésive l'ensemble générateur de micro-courants (30) au sujet.

2. Système médical (10) selon la revendication 1, dans lequel l'ensemble générateur de micro-courants (30) possède une ouverture (31) dont la taille est adaptée à la taille d'une plaie ou d'une zone cutanée (2) à traiter, et dans lequel les électrodes (32A, 32B) de la paire d'électrodes sont disposées sur les côtés opposés de l'ouverture (31).

3. Système médical (10) selon la revendication 1, dans lequel la paire d'électrodes de l'ensemble générateur de micro-courants (30) comporte une électrode externe (32D) et une électrode interne (32C) disposée à l'intérieur de l'électrode externe (32D).

4. Système médical (10) selon l'une quelconque des revendications précédentes, dans lequel l'ensemble générateur de champ magnétique (20) a une structure en couches et comprend deux bobines électromagnétiques (24A, 24B), et dans lequel une bobine électromagnétique (24A) dans une couche spirale radialement vers l'intérieur, et l'autre bobine électromagnétique (24B) dans une autre couche spirale radialement vers l'extérieur, et dans lequel les deux bobines électromagnétiques (24A, 24B) sont reliées électriquement l'une à l'autre.

5. Système médical (10) selon la revendication 1, dans lequel la couche adhésive (36) est constituée d'un matériau hydrogel biocompatible ou d'un polyuréthane thermoplastique.

6. Système médical (10) selon la revendication 1 ou 5, dans lequel la paire d'électrodes (32A, 32B) est imprimée sur la couche adhésive (36) à l'aide de la technologie PCB.

7. Système médical (10) selon la revendication 1 ou 5, dans lequel la couche adhésive (36) comporte des vides dans lesquels la paire d'électrodes (32A, 32B) est disposée.

8. Système médical (10) selon l'une quelconque des revendications précédentes, dans lequel l'au moins une bobine électromagnétique (24) est une bobine électromagnétique plane.

9. Système médical (10) selon l'une quelconque des revendications précédentes, dans lequel l'au moins une bobine électromagnétique (24) est imprimée sur une couche électriquement isolante (25) ou sur une couche supérieure transparente (27) à l'aide de la technologie PCB.

10. Système médical (10) selon l'une quelconque des revendications précédentes, dans lequel chaque électrode (32A, 32B) de la paire d'électrodes et l'au moins une bobine électromagnétique (24) possède des embouts configurés pour être reliés à un générateur de signaux (100) configuré pour piloter électriquement la paire d'électrodes (32A, 32B) et l'au moins une bobine électromagnétique (24).

11. Système médical (10) selon l'une quelconque des revendications précédentes, comprenant en outre une puce d'identification programmée pour permettre l'identification du système médical (10).

12. Système médical (10) selon l'une quelconque des revendications précédentes, comprenant en outre un capteur possédant des embouts configurés pour être reliés à un enregistreur de données.

13. Système médical (10) selon l'une quelconque des revendications précédentes, dans lequel l'ensemble générateur de micro-courants (30) est configuré pour générer des micro-courants dans une plage de 0 à 30 mA, de préférence 0 à 10 mA, et dans lequel l'ensemble générateur de champ magnétique (20) est configuré pour générer un champ magnétique avec un flux magnétique dans une plage de 0 à 30 mT, de préférence 0 à 10 mT.

14. Système médical (10) selon l'une quelconque des revendications précédentes, dans lequel le moyen d'engagement (40) est configuré de manière à ce qu'une distance entre l'au moins une bobine électromagnétique (24) et le patient soit comprise entre 1 et 10 mm, de préférence 2 mm.

15. Système médical (10) selon l'une quelconque des revendications précédentes, dans lequel le moyen d'engagement (40) est une liaison mécanique à ajustement de forme entre l'ensemble générateur de micro-courants (30) et l'ensemble générateur de champ magnétique (20).

16. Système médical (10) selon l'une quelconque des revendications précédentes, dans lequel le moyen d'engagement comporte un collier (40) prévu sur l'ensemble générateur de micro-courants (30) et une ouverture (21) prévue sur l'ensemble générateur de champ magnétique (20), et dans lequel l'ouverture (21) est dimensionnée de telle sorte que le collier (40) de l'ensemble générateur de micro-courants (30) s'adapte de manière ajustée en forme dans à l'ouverture (21) de l'ensemble générateur de champ magnétique (20).

17. Système médical (10) selon l'une quelconque des revendications 1 à 14, dans lequel le moyen d'engagement est une charnière (40') qui permet un mouvement pivotant de l'ensemble générateur de champ magnétique (20) par rapport à l'ensemble générateur de micro-courants (30).

18. Système médical (10) selon la revendication 17, dans lequel la charnière (40') est une charnière mobile.
